# DEMANDE DE BREVET EUROPEEN

(11) **EP 4 375 363 A1**
(43) Date de publication de la demande: **29.05.2024**
(21) Numéro de dépôt: 23211917.2
(22) Date de dépôt: 24.11.2023
(51) Int. Cl.: C12M 1/34

(54) **SYSTÈME DE CAPTEUR GÉNÉRIQUE ET STÉRILE SUR MÂT MOBILE**

(30) Priorité: 25.11.2022 FR 2212356
(71) Demandeur: COMMISSARIAT À L'ÉNERGIE ATOMIQUE ET AUX ÉNERGIES ALTERNATIVES, 75015 Paris (FR); Global Process Concept, 17180 Perigny (FR)
(72) Inventeur: CAROFF, Tristan, 38054 GRENOBLE CEDEX 09 (FR); BRULAIS, Sébastien, 38054 GRENOBLE CEDEX 09 (FR); GAUROY, Martin, 38054 GRENOBLE CEDEX 09 (FR); BURLET, Jean-Yves, 38054 GRENOBLE CEDEX 09 (FR); GARAFFA, Clément, 38054 GRENOBLE CEDEX 09 (FR); POPSE, Zsolt, 17180 PERIGNY (FR)
(74) Mandataire: Atout PI Laplace

(57) **Abrégé**

L'invention concerne un dispositif d'analyse (1) d'au moins un paramètre d'une réaction (2) chimique ou biochimique ou biologique dans une enceinte réactionnelle (3), le dispositif d'analyse (1) comprenant:
- un bras (4) destiné à être fixé selon une liaison glissière (41) contre une face (300) d'une paroi (30) de l'enceinte réactionnelle (3) par l'intermédiaire d'une extrémité fixe (40), le bras (4) ayant un degré de liberté en translation par rapport à la paroi (30) ;
- un capteur (5) configuré pour mesurer l'au moins un paramètre, le capteur (5) étant fixé à une extrémité libre (42) du bras (4) ;
- un dispositif de motorisation (7) configuré pour induire un déplacement axial du bras (4) selon le degré de liberté ;
- un dispositif d'isolation hermétique (8) du bras (4) par rapport à la réaction (2), le dispositif d'isolation hermétique (8) reliant le bras (4) à la face (300) de la paroi (30).

## Description

L'invention se rapporte aux domaines de l'industrie pharmaceutique, de la cosmétique, de la production biomasse ou encore de l'agroalimentaire.

En particulier, l'invention concerne un dispositif d'analyse et de suivi d'au moins un paramètre d'une réaction chimique ou biochimique ou biologique dans une enceinte réactionnelle.

La multiplication ou la culture des micro-organismes est largement mise en oeuvre dans l'industrie pharmaceutique, la cosmétologie ou encore l'industrie agroalimentaire pour la production de biomasse, pour la production d'un métabolite ou encore la bioconversion d'une molécule d'intérêt, mais également la vinification et le brassage.

Cette multiplication est avantageusement exécutée dans un bioréacteur qui permet d'exercer un contrôle des conditions de culture, et plus particulièrement des paramètres réactionnels tels que la température, le pH, le niveau d'oxygène ou encore le taux de croissance de la biomasse.

De manière connue, un bioréacteur comprend une enceinte réactionnelle dans laquelle sont généralement disposés un agitateur et/ou un aérateur destinés à, respectivement, homogénéiser et oxygéner le milieu réactionnel. Le bioréacteur comprend en outre une enveloppe thermique qui permet d'imposer une thermalisation au milieu réactionnel. Un tel bioréacteur est également pourvu de capteurs, par exemple des capteurs de température, de pH, d'oxygène dissous, afin d'assurer un suivi de l'état de la culture, et, le cas échéant, ajuster, de préférence en temps réel, les conditions de culture au sein de l'enceinte réactionnelle.

Or, ces capteurs sont généralement montés de manière fixe sur des tiges plongées dans le milieu réactionnel.

Cependant, cette fixation de capteur présente de nombreux inconvénients.

En effet, les capteurs maintenus fixes ne sondent le milieu réactionnel que localement, c'est-à-dire à proximité des capteurs mêmes, et ne détectent pas toute potentielle inhomogénéité dans le milieu réactionnel se produisant à distance des capteurs. Les données collectées par ces capteurs ne permettent alors pas de procéder à un ajustement optimal des paramètres réactionnels.

Dès lors, afin de mieux rendre compte de l'inhomogénéité du milieu réactionnel, il a été envisagé de considérer un « capteur mobile ». Un tel capteur peut notamment être logé dans une capsule plongée dans le milieu réactionnel. La capsule est alors soumise aux courants imposés par l'agitateur et est ainsi exposée à différentes zones du milieu réactionnel.

Néanmoins, ces capsules présentent l'inconvénient, dans la mesure où leur déplacement dans le bioréacteur est libre, d'entrer régulièrement en contact avec les parois du bioréacteur ou des pales de l'agitateur, et par conséquent d'être endommagées.

Par ailleurs, le parcours des capsules dans le milieu réactionnel reste aléatoire, et ne permet pas de couvrir toute l'étendue du bioréacteur.

Ainsi, il a été envisagé, comme divulgué dans le document EP 3 967 743 A1 de contrôler le mouvement du capteur mobile par l'intermédiaire d'un mât fixé contre deux parois opposées de l'enceinte réactionnelle. Le kit d'analyse décrit dans le document EP 3 967 743 A1 propose ainsi un mât creux fixé contre deux parois opposées de l'enceinte réactionnelle contre laquelle un capteur mobile contre ce mât et fixé par l'intermédiaire d'une liaison magnétique au mât. Le capteur est alors mobile en translation selon un axe transversal fermé par le mât.

Cependant, une telle installation présente de nouveaux inconvénients. En effet, il existe deux principaux types de bioréacteurs : les bioréacteurs en inox ou en verre et les bioréacteurs souples à usage unique (« SUB » pour *Single Use Bioreactor).* La principale différence est alors que le bioréacteur en inox ou en verre est utilisable un grand nombre de fois et nécessite donc un nettoyage complexe (acide/Base) afin d'éliminer tout organisme avant la production suivante alors que les bioréacteurs souples à usage unique sont jetables après une seule utilisation et présentent l'avantage de garantir la stérilité sans nettoyage. Les bioréacteurs souples à usage unique représentent aujourd'hui environ 80 % de la production pour l'industrie pharmaceutique. Ces bioréacteurs jetables présentent une structure porteuse moins rigide en comparaison des bioréacteurs en inox ou en verre.

Dès lors, les bioréacteurs jetables sont généralement non adaptés pour recevoir un tel dispositif comme divulgué dans le document EP 3 967 743 A1. Plus précisément, les parois des enceintes réactionnelles des bioréacteurs souples à usage unique ne sont pas adapté à la double fixation du mât et nécessitent donc d'être adaptées pour pouvoir incorporer le mât. Il est alors complexe d'adapter un tel dispositif d'analyse pour des bioréacteurs souple à usage unique avec l'ajout d'une fixation en haut de l'enceinte réactionnelle et une fixation en bas de l'enceinte réactionnelle.

En outre, un tel dispositif présente des problèmes de stérilité lors de l'insertion du système dans le bioréacteur.

L'invention vise à pallier tout ou partie des problèmes cités plus haut en proposant un système de capteur générique et stérile intégré sur un mât mobile pour la mesure de grandeurs physiologiques ou physique sur l'ensemble de la hauteur d'un bioréacteur, le mât étant facilement intégrable sur toute enceinte réactionnelle. Le dispositif d'analyse selon l'invention présente également l'avantage de permettre d'introduire le capteur et le mât mobile de manière hermétique et sans impacter le milieu réactionnel.

A cet effet, l'invention a pour objet un dispositif d'analyse d'au moins un paramètre d'une réaction chimique ou biochimique ou biologique dans une enceinte réactionnelle, le dispositif d'analyse comprenant:
- un bras destiné à être fixé selon une liaison glissière contre une face d'une paroi de l'enceinte réactionnelle par l'intermédiaire d'une extrémité fixe, le bras ayant un degré de liberté en translation par rapport à la paroi de l'enceinte réactionnelle ;
- un capteur configuré pour mesurer l'au moins un paramètre, le capteur étant fixé à une extrémité libre du bras ;
- un dispositif de motorisation configuré pour induire un déplacement axial du bras selon le degré de liberté ;
- un dispositif d'isolation hermétique du bras par rapport à la réaction chimique ou biochimique ou biologique, le dispositif d'isolation hermétique reliant le bras à la face de la paroi de l'enceinte réactionnelle.

Selon un aspect de l'invention, le dispositif d'isolation hermétique comprend un matériau parmi le silicone, un élastomère fluoré, le polyétheréthercétone, l'éthylène-propylène-diène monomère, du polytétrafluoroéthylène.

Selon un aspect de l'invention, le dispositif d'isolation hermétique comprend un soufflet.

Selon un aspect de l'invention, la paroi comprend une face interne en regard de la réaction, le dispositif d'isolation hermétique étant disposé de sorte à relier le bras à la face interne de la paroi.

Selon un aspect de l'invention, la paroi comprend une face externe en regard d'un milieu extérieur à la réaction, le dispositif d'isolation hermétique étant disposé de sorte à relier le bras à la face externe de la paroi.

Selon un aspect de l'invention, le dispositif d'analyse comprend un moyen de commande du bras, connecté au dispositif de motorisation et agencé pour commander le déplacement du bras selon le degré de liberté.

Selon un aspect de l'invention, le dispositif d'analyse comprend un moyen de transmission de données susceptibles d'être mesurées par le capteur.

Selon un aspect de l'invention, le dispositif d'analyse comprend un dispositif d'enregistrement des données mesurées par le capteur.

Selon un aspect de l'invention, le capteur est un capteur apte à mesurer une donnée parmi le pH, l'oxygène dissout, le dioxyde de carbone, la conductivité électrique, le potentiel redox ou la température.

Selon un aspect de l'invention, le bras est un bras télescopique.

Selon un aspect de l'invention, le dispositif d'isolation hermétique comprend un joint positionné entre le soufflet et la face de la paroi de l'enceinte réactionnelle, le joint comprenant une première surface d'étanchéité en contact avec la face de la paroi de l'enceinte réactionnelle et une deuxième surface d'étanchéité en contact avec le soufflet.

L'invention a également trait à une enceinte réactionnelle d'une réaction chimique ou biochimique ou biologique comprenant le dispositif d'analyse d'au moins un paramètre de la réaction chimique ou biochimique ou biologique dans l'enceinte réactionnelle.

L'invention sera mieux comprise et d'autres avantages apparaîtront à la lecture de la description détaillée d'un mode de réalisation donné à titre d'exemple, description illustrée par le dessin joint dans lequel :
[Fig.1] la figure 1 représente une vue schématique d'un dispositif d'analyse et de suivi selon la présente invention ;
[Fig.2] la figure 2 représente une vue schématique du dispositif d'analyse et de suivi dans une architecture préférentielle ;
[Fig.3] la figure 3 représente une vue schématique d'une variante du dispositif d'analyse et de suivi de la figure 2 ;
[Fig.4] la figure 4 représente une vue schématique d'une enceinte réactionnelle comprenant deux dispositif d'analyse selon l'invention ;
[Fig.5A] la figure 5A représente une vue schématique du dispositif d'analyse et de suivi selon un premier mode de configuration dans lequel un dispositif d'isolation hermétique est étendu ;
[Fig.5B] la figure 5B représente une vue schématique du dispositif d'analyse et de suivi de la figure 5A dans lequel le dispositif d'isolation hermétique est comprimé ;
[Fig.6A] la figure 6A représente une vue schématique du dispositif d'analyse et de suivi selon un deuxième mode de configuration dans lequel le dispositif d'isolation hermétique est étendu ;
[Fig.6B] la figure 6B représente une vue schématique du dispositif d'analyse et de suivi de la figure 6A dans lequel le dispositif d'isolation hermétique est comprimé ;
[Fig.7] la figure 7 représente une vue détaillé du dispositif d'analyse et de suivi des figures 6A et 6B ;
[Fig.8] la figure 8 représente une vue détaillée de la fixation du bras du dispositif d'analyse et de suivi selon l'invention,
[Fig. 9] la figure 9 représente une vue agrandie d'une face de l'enceinte hermétique contre laquelle repose le dispositif d'isolation hermétique selon l'invention.

Par souci de clarté, les mêmes éléments porteront les mêmes repères dans les différentes figures.

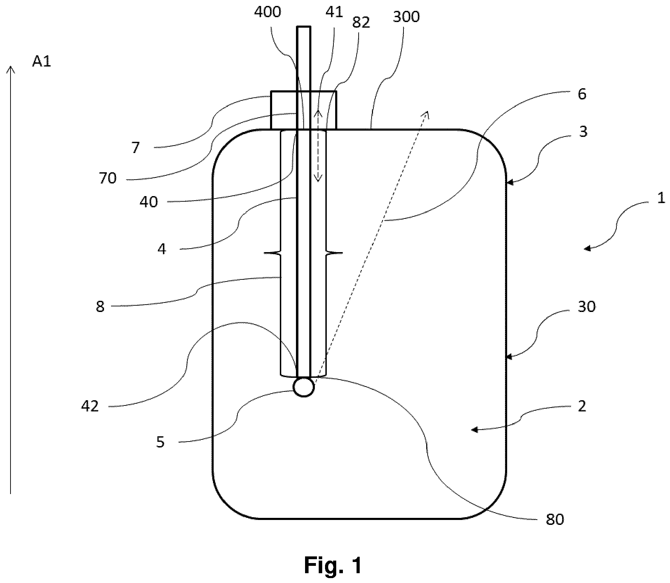

La figure 1 représente une vue schématique d'un dispositif d'analyse et de suivi 1 d'au moins un paramètre d'une réaction chimique ou biochimique ou biologique 2 dans une enceinte réactionnelle 3.

Le dispositif d'analyse 1 comprend un bras 4 destiné à être fixé selon une liaison glissière 41 contre une face 300 d'une paroi 30 de l'enceinte réactionnelle 3 par l'intermédiaire d'une extrémité fixe 40. Le bras 4 a un degré de liberté en translation par rapport à la paroi 30 de l'enceinte réactionnelle 3. Autrement dit, le bras 4 est configuré pour s'étendre axialement par rapport à la paroi 30 et localement par rapport à la face 300 selon un premier axe A1. Selon un aspect préférentiel de l'invention, le bras 4 translate sensiblement perpendiculairement par rapport à la face 300 de la paroi 30.

En variante, le bras 4 est apte à translater selon un axe sécant par rapport à la paroi 300. Cet axe de translation peut être quelconque tant que le bras n'entre pas en contact de manière non souhaitée avec un composant comme l'agitateur du bioréacteur.

Le bras 4 est donc en liaison glissière avec la face 300 de la paroi 30 de l'enceinte réactionnelle 3 de manière à pouvoir être guidé selon le premier axe A1 en translation.

Le dispositif d'analyse 1 comprend également un capteur 5 configuré pour mesurer l'au moins un paramètre de la réaction 2, le capteur 5 étant fixé à une extrémité libre 42 du bras 4 au contact de la réaction 2. Cette extrémité libre 42 est une extrémité mouvante grâce à la mobilité en translation du bras 4. L'extrémité libre 42 est, par conséquent, sensiblement opposée à la face 300 de la paroi 30. En outre, le capteur 5 est donc destiné à collecter des données d'un milieu réactionnel dans lequel le capteur 5 est susceptible d'être plongée.

Le dispositif d'analyse 1 pet également comprendre plusieurs capteurs 5 fixés à l'extrémité libre 42 du bras 4 de sorte à mesurer plusieurs paramètres de la réaction 2.

Notamment, les données collectées par le ou les capteurs 5 peuvent être relatives aux conditions expérimentales imposées au milieu réactionnel. Ces dernières peuvent être caractéristiques de l'acidité, de l'agitation, de la température. Les données collectées peuvent également être relatives à l'avancement de la réaction 2 ou des phénomènes mis en jeu dans le milieu réactionnel.

À titre d'exemple, le ou les capteurs 5 peuvent comprendre au moins un des éléments choisis parmi: un capteur de mesure de pH, un capteur de mesure de température, un capteur de mesure d'une quantité d'oxygène dissous, un capteur de mesure d'une quantité de dioxyde de carbone ou d'oxygène dissout, un capteur apte à mesurer la conductivité électrique ou le potentiel redox, c'est-à-dire la réaction d'oxydo-réduction se produisant au sein de la réaction 2. L'invention n'est pas limitée à ces seuls capteurs, et l'homme du métier, en fonction des paramètres réactionnels à suivre, pourra mettre en oeuvre tout autre capteur.

Le dispositif d'analyse 1 comprend également un moyen de transmission de données 6 susceptibles d'être mesurées par le capteur 5. Le moyen de transmission de données 6 permet donc de transmettre toutes les données collectées par le capteur 5 vers une plateforme permettant l'analyse en temps réel de l'évolution du milieu réactionnel.

De plus, le dispositif d'analyse 1 comprend un dispositif de motorisation 7 configuré pour induire le déplacement axial du bras 4 selon le degré de liberté. Autrement dit, le dispositif de motorisation 7 permet d'induire la translation du bras 4 et donc du capteur 5 selon le premier axe A1 dans l'enceinte réactionnelle 3. En outre, le dispositif de motorisation 7 peut, selon une configuration préférentielle, comprendre un canal de guidage 70 du bras 4 traversé par le bras 4 lors de ses translations selon le premier axe A1 permettant de canaliser le mouvement du bras 4.

Le dispositif de motorisation 7 permet ainsi de mesurer, par le biais du capteur 5, le paramètre de la réaction 2 chimique ou biochimique ou biologique dans l'enceinte réactionnelle 3. Cette mesure peut avantageusement se faire selon différents niveaux ou différentes hauteurs dans l'enceinte réactionnelle 3 grâce au dispositif de motorisation 7 qui permet des translations maitrisées du bras 4 et du capteur 5. Le dispositif de motorisation peut être, de façon préférentielle, un actionneur linéaire à courroie. Ce type d'actionneur présente l'avantage de permettre de permettre de contrôler les translations du bras 4 et du capteur 5 de sorte à permettre au capteur 5 de mesurer un paramètre dans la réaction 2 à différentes positions, différents niveaux ou différentes hauteurs dans l'enceinte réactionnelle 3.

En variante, il peut également être envisagé que le bras 4 soit guidé lors de ses translations par le biais d'un double cardan ou une connectique souple de type silentbloc afin de ne pas sur contraindre le mouvement de translation. Cet ensemble permet de conserver un bon alignement du bras 4, de résister à la charge radiale dans la liaison entre le bras 4 et le dispositif de motorisation 7 due à la pression de la réaction 2 sur le bras 4 et garantir une étanchéité entre l'intérieur du dispositif d'isolation hermétique 8 et la réaction 2 afin d'éviter toute contamination.

A titre d'exemple indicatif, le dispositif de motorisation 7 peut être un moteur par vis à bille, pantographe ou un actionneur linéaire.

En variante, tout dispositif motorisé permettant d'induire un mouvement de translation peut être envisagé.

En outre, le dispositif d'analyse 1 comprend un dispositif d'isolation hermétique 8 du bras 4 par rapport à la réaction 2 chimique ou biochimique ou biologique. Le dispositif d'isolation hermétique 8 est disposé de sorte à relier le bras 4 à la face 300 de la paroi 30 de l'enceinte réactionnelle 3. Le dispositif d'isolation hermétique 8 permet donc d'isoler le bras 4 de la réaction 2 dans l'enceinte réactionnelle 3. Dès lors, la stérilité de la réaction 2 est garanti même lorsque le bras 4 est en mouvement dans l'enceinte réactionnelle 3.

Selon l'invention, le dispositif d'isolation hermétique 8 est disposé de sorte à empêcher toute introduction d'un corps étranger à la réaction 2 par une ouverture 400 formée au niveau de l'extrémité fixe 40 lors de la translation du bras 4. En effet, lorsque le bras 4 subit une translation en direction d'une face opposée à la face 300 de la paroi 30, traduisant alors un mouvement d'introduction du bras 4 et du capteur 5 dans l'enceinte réactionnelle 3, il est possible qu'un corps étranger à la réaction 2 se fixe au bras 4 et traverse la paroi 30 de l'enceinte réactionnelle 3 au niveau de l'ouverture 400 et se retrouve en contact du milieu réactionnel et contamine la réaction 2 en cours. Dès lors, le dispositif d'isolation hermétique 8 assure une zone d'étanchéité entre le bras 4 et la réaction 2 en toute situation de translation selon le premier axe A1.

Selon une configuration préférentielle, le dispositif d'isolation hermétique 8 comprend une première extrémité étanche 80 reliée à l'extrémité libre 42 et une deuxième extrémité étanche 82 reliée à la face 300 de la paroi 30 aux alentours de l'extrémité fixe 40 par exemple.

Selon l'invention, le dispositif d'isolation hermétique 8 comprend un matériau parmi le silicone, un élastomère fluoré, le polyétheréthercétone (PEEK), l'éthylène-propylène-diène monomère (EPDM), du polytétrafluoroéthylène (PTFE) ou dans tout autre matériau biocompatible avec la réaction 2 afin de ne pas impacter le milieu réactionnel.

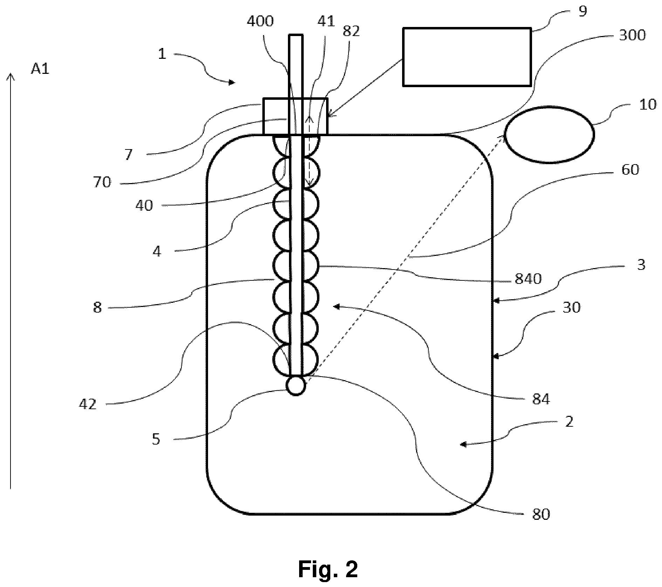

Selon une configuration préférentielle, le dispositif d'isolation hermétique 8 comprend un soufflet 84, comme représenté en figure 2. Le soufflet 84 présente l'avantage d'être adapté à chaque situation du bras 4. En effet, un soufflet 84 présente une structure élastique et extensible en forme de boursouflure 840 permettant d'être tendue en étendant chaque boursouflure 840 et d'être comprimée en compressant chaque boursouflure 840. L'utilisation d'un soufflet 84 adapté au mouvement du bras 4 présente ainsi l'avantage de ne pas impacter la réaction 2 lors des déplacements du bras 4 et du capteur 5, par exemple en générant des perturbations locales et des mouvements de courant dans la réaction 2 non souhaités. En outre, un tel type de soufflet 84 présente l'avantage d'être résistant à la multitude de translations du bras et donc à l'effort en traction et en compression qui lui est soumis.

A titre d'exemple indicatif, le soufflet 84 peut être de section cylindrique, rectangulaire ou conique.

En variante, le dispositif d'isolation hermétique 8 comprend une poche cylindrique élastique apte à subir l'effort en traction et en compression exercé par la translation du bras 4.

De manière avantageuse, le dispositif d'analyse 1 peut également comprendre un moyen de commande 9 du bras 4 agencé pour commander le déplacement du bras 4 selon le degré de liberté. Plus précisément, le moyen de commande 9 est connecté au dispositif de motorisation 7 de sorte à ce que le dispositif de motorisation 7 induise une translation du bras 4 et du capteur 5 selon le premier axe A1 en réponse à une commande du moyen de commande 9. Le moyen de commande 9 assure donc un pilotage du bras 4. A titre d'exemple indicatif, la vitesse linéaire de translation typique du bras 4 est de l'ordre de 0,01 m/s à 1,5 m/s et la résolution spatiale est de l'ordre de 0,1 mm à 1 cm selon le type de moteur ou d'actionneur linéaire utilisé comme dispositif de motorisation 7.

Le moyen de commande 9 peut alors permettre de configurer de manière prédéfinie la ou les translations successives du bras 4 dans l'enceinte réactionnelle 3 ou être commandé en temps réel par un utilisateur.

Le dispositif d'analyse 1 peut également comprendre un dispositif d'enregistrement 10 des données mesurées par le capteur 5. Le dispositif d'enregistrement 10 communique alors avec le capteur 5 par le biais du moyen de transmission de données 6. En outre, le moyen de transmission de données 6 peut être un moyen de transmission sans fil 60 de type wifi, ZigBee ou Bluetooth par exemple.

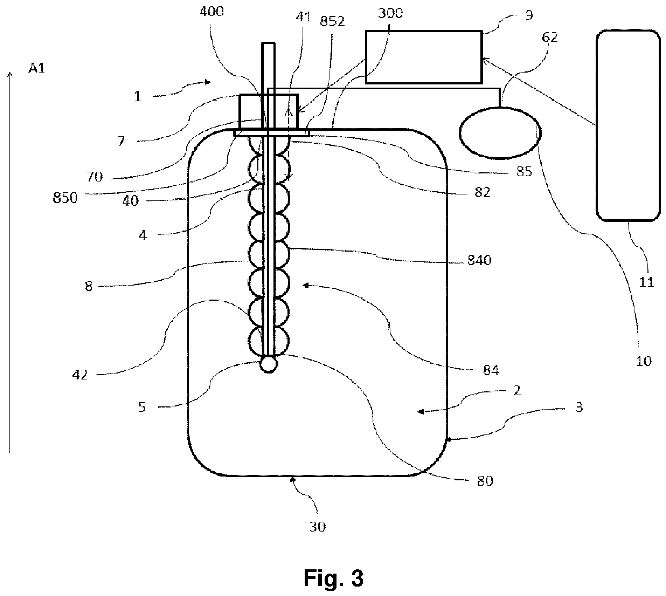

En variante, comme représenté en figure 3, le moyen de transmission de données 6 peut être un moyen de transmission filaire 62 reliant le capteur 5 au dispositif d'enregistrement 10 de type RS432, RS485, ou RJ45 par exemple.

Le dispositif d'analyse 1 peut également comprendre une source d'alimentation 11 configurée pour alimenter le moyen de commande 9 et le dispositif de motorisation 7.

De manière avantageuse, le dispositif d'isolation hermétique 8 peut également comprendre un joint 85 positionné entre le soufflet 84 ou la poche cylindrique et la face 300 de la paroi 30 de l'enceinte réactionnelle 3. En effet, le joint 85 comprend une première surface d'étanchéité 850 en contact avec la face 300 de la paroi 30 de l'enceinte réactionnelle 3 et une deuxième surface d'étanchéité 852 en contact avec le soufflet 84 ou avec la poche cylindrique. Le joint 85 assure ainsi une isolation supplémentaire au niveau de l'ouverture 400.

En effet, le fait de translater le bras 4 et plus précisément, de rentrer et sortir le bras 4 du milieu réactionnel 2 de l'enceinte réactionnelle est très risqué en termes de contamination pour la réaction 2. Le joint 85 empêche alors le soufflet 84 ou la poche cylindrique d'être en contact de la réaction 2 réduisant alors le risque de contamination. Autrement dit, le joint 85 permet de générer un espace intermédiaire ou volume intermédiaire 800 entre l'enceinte réactionnelle 3, qui contient la réaction 2 et le milieu extérieure à l'enceinte réactionnelle 3. Comme représenté en figure 1 ou en figure 2, ce volume intermédiaire 800 est défini comme le volume interne au soufflet 84 ou à la poche cylindrique. Il s'agit du volume compris entre le soufflet 84 ou la poche cylindrique et le bras 4. Ce volume intermédiaire 800, compris dans le soufflet 84 ou la poche cylindrique, n'est jamais en contact avec la réaction 2. Ce volume intermédiaire 800 compris dans le soufflet 84 présente ainsi l'intérêt de sécuriser la réaction 2 par rapport à toute contamination extérieure à l'enceinte réactionnelle 3. Tout agent contaminant externe à l'enceinte réactionnelle 3 est alors bloqué par le joint 85 dans le volume intermédiaire 800 compris dans le soufflet 84 ou la poche cylindrique.

Ainsi, l'utilisation du joint 85 permet des mesures du capteur 5 à différents niveaux dans l'enceinte réactionnelle 3 et à différentes positions par l'actionnement du dispositif de motorisation 7. Le dispositif de motorisation et le joint 85 permettent ainsi de faire translater le bras 4 et le capteur 5 sans risque une quelconque contagion pour la réaction 2.

En variante, le joint 85 peut être un joint racleur comprenant une lèvre interne flexible en appui contre le bras 4 et une lèvre externe fixée à la paroi 30 de l'enceinte réactionnelle 3. Plus précisément, la lèvre interne est serrée contre le bras 4 de sorte à assurer une étanchéité de la part du joint racleur lors du coulissage du bras 4. En outre, le joint racleur peut également comprendre plusieurs lèvres internes superposées et serrées contre le bras 4 pour améliorer l'étanchéité du joint racleur.

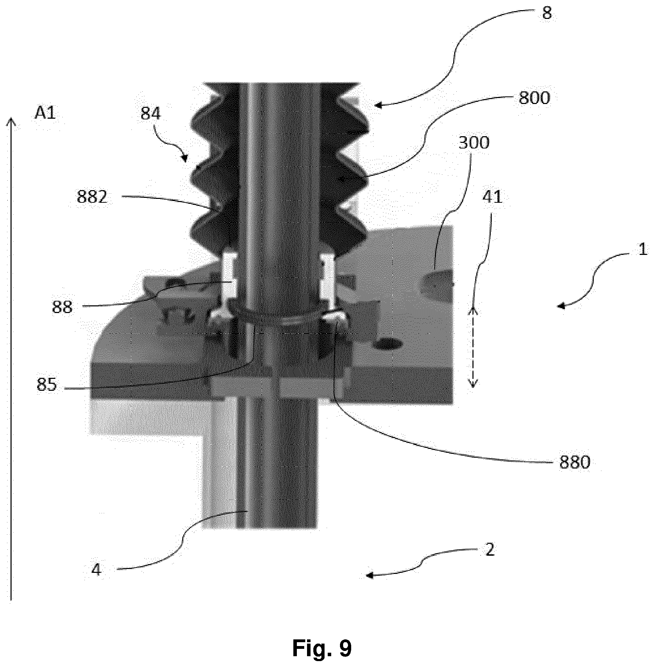

Le dispositif d'isolation hermétique 8 peut également comprendre un élément de guidage 88, représenté en figure 9. L'élément de guidage 88 est en appui sur la face 300 selon un premier bord 880 de l'élément de guidage 88. L'élément de guidage 88 comprend une section 882 de forme complémentaire du bras 4. Dans l'exemple illustré, le bras 4 présente une forme cylindrique. L'élément de guidage comprend alors une section ouverte 882 de forme cylindrique de dimension complémentaire du bras 4. Le bras 4 traverse l'élément de guidage 88 et la face 300 lors de ses translations guidées par le dispositif de motorisation 7. Le bras 4 traverse l'élément de guidage 88 selon la liaison glissière 41 par rapport au premier axe A1. L'élément de guidage 88 est configuré pour guider la translation du bras 4 parallèlement au premier axe A1 de sorte que la translation du bras 4 par le dispositif de motorisation 7 est exactement parallèle au premier axe A1. Grâce à l'élément de guidage 88, il n'existe pas de mouvement relatif du bras 4 par rapport à la face 300 dans le plan parallèle à la face 300 de la paroi 30. Le seul mouvement entre le bras 4 et la face 300 est alors le mouvement de translation selon le premier axe A1 dû à la liaison glissière 41 entre la face 300 et le bras 4. L'élément de guidage 88 est donc en contact du bras 4 par sa section ouverte 882 traversée par le bras 4 et guide la translation du bras 4.

Le joint 85 bloque les contaminants extérieurs de sorte qu'aucun contaminant ne peut pénétrer dans l'enceinte réactionnelle 3 et l'ensemble de guidage 88 maintient le bras 4 centré selon le premier axe A1 de manière précise, ce qui améliore les performances du système d'étanchéité. En effet, le fait de n'avoir aucun mouvement relatif entre le bras 4 et la face 300 dans le plan parallèle à la face 300 permet de n'avoir aucune déformation du joint 85 dans le plan parallèle à la face 300, c'est-à-dire des déformations radiales. Or, ces déformations radiales peuvent limiter les capacités d'étanchéité du joint 85. Bloquer ainsi les mouvements relatifs entre le bras 4 et la face 300 permet alors d'améliorer l'étanchéité du dispositif d'isolation hermétique 8.

L'élément de guidage 88 peut être obtenu en Polyether Ether Ketone (PEEK) qui est un thermoplastique rigide et résistant aux rayons gamma.

Le joint 85 peut être obtenu dans en éthylène-propylène-diène monomère (EPDM) qui présente une bonne élasticité.

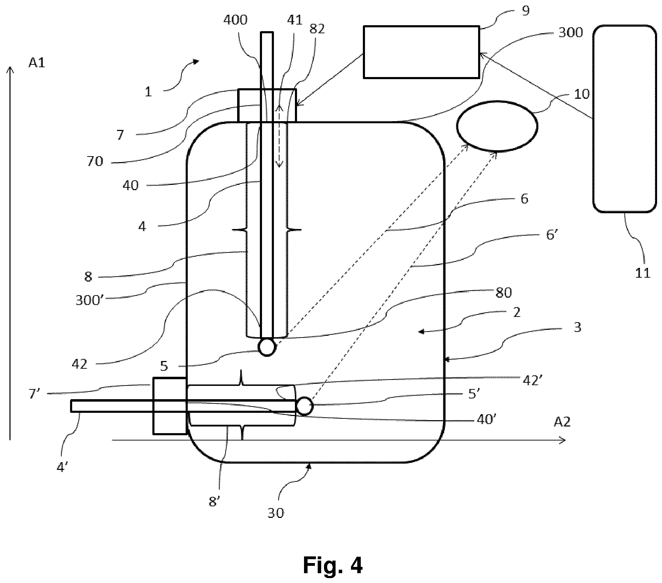

Il peut également être envisagé, comme représenté en figure 4, d'utiliser plusieurs dispositif d'analyse 1. En effet, l'utilisation d'un bras 4 capable de translater selon le premier axe A1, limite l'acquisition de données du capteur 5 à une gamme de hauteur, dans le cas illustré, sans pouvoir observer le milieu réactionnel selon un deuxième axe A2 au moins sécant et préférentiellement perpendiculaire au premier axe A1 selon un plan formé par le premier axe A1 et le deuxième axe A2.

Le dispositif d'analyse 1 peut alors comprendre :
- un deuxième bras 4' destiné à être fixé sur une deuxième face 300' de la paroi 30 de l'enceinte réactionnelle 3 par l'intermédiaire d'une extrémité fixe 40'. Le deuxième bras 4' possède également un degré de liberté en translation par rapport à la paroi 30 de l'enceinte réactionnelle 3. Dès lors, le deuxième bras 4' translate selon le deuxième axe A2.
- un deuxième capteur 5' configuré pour mesurer au moins un paramètre, fixé à une extrémité libre 42' du deuxième bras 4',
- un deuxième moyen de transmission 6' de données susceptibles d'être mesurées par le capteur 5' du deuxième bras 4',
- un deuxième dispositif de motorisation 7' configuré pour induire un déplacement axial du deuxième bras 4' selon le degré de liberté du deuxième ras 4' et donc selon le deuxième axe A2,
- et un deuxième dispositif d'isolation hermétique 8' du deuxième bras 4' par rapport à la réaction 2 chimique ou biochimique ou biologique, configuré pour relier le deuxième bras 4' à la deuxième face 300' de la paroi 30 de l'enceinte réactionnelle 3.

La première face 300 et la deuxième face 300' sont deux faces différentes de la paroi 30 de l'enceinte réactionnelle 3 et, préférentiellement, sont deux faces s'étendant de manière perpendiculaire ou au moins sécante.

En variante, le deuxième axe A2 peut également être sécant au premier axe A1 dans le plan formé par le premier axe A1 et par le deuxième axe A2.

Afin de ne pas limiter les mouvements du bras 4 et du deuxième bras 4', il est souhaité que le bras 4 et le deuxième bras 4' soient distants l'un de l'autre selon un axe perpendiculaire au premier axe A1 et au deuxième axe A2.

En variante, il peut être envisagé d'utiliser plus de deux bras ayant un degré de liberté en translation dans le dispositif d'analyse 1.

Dans la suite de la description, un seul bras 4 est mentionné. Néanmoins, il peut être envisagé d'utiliser également le deuxième bras 4' voire plus de deux bras.

Le bras 4 peut avantageusement être un bras télescopique. Un bras télescopique présente l'avantage d'être compact.

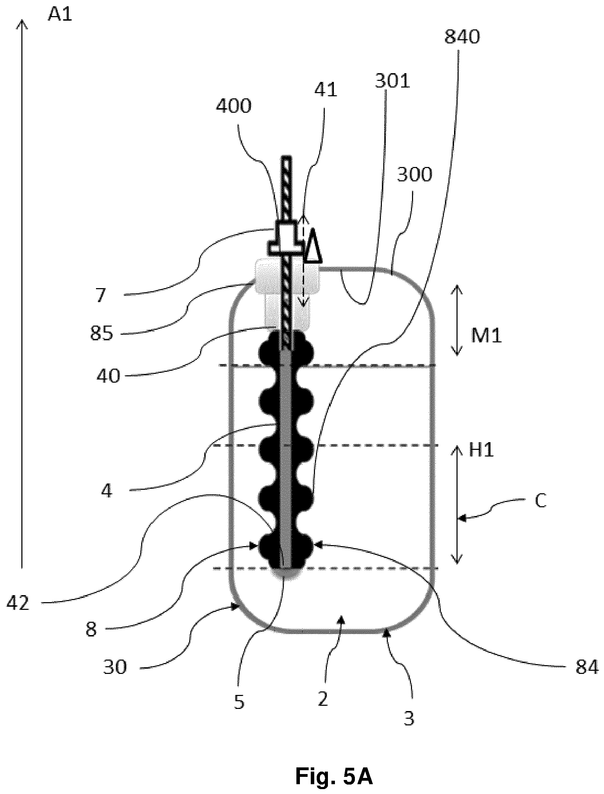

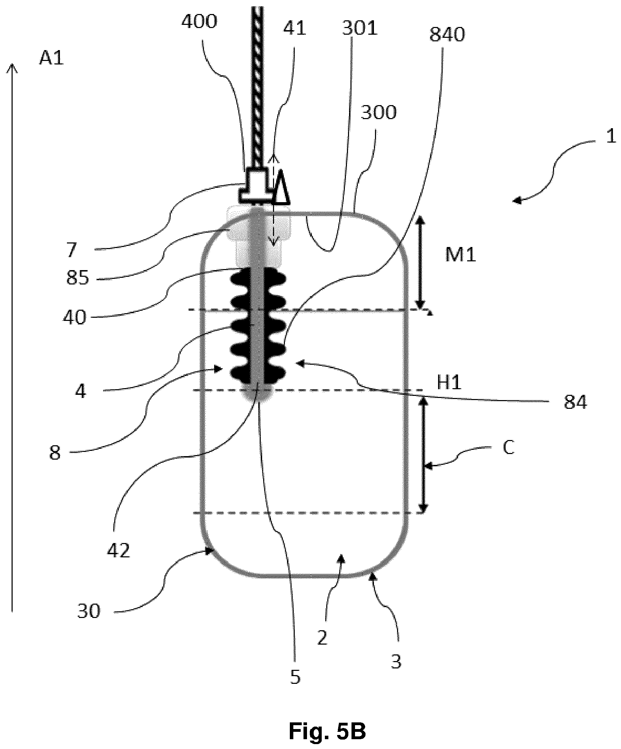

Selon un premier mode de configuration représenté en figure 5A et 5B, le dispositif d'isolation hermétique 8 peut être compris à l'intérieur de l'enceinte réactionnelle 3. Dès lors, le dispositif d'isolation hermétique 8, selon cette première configuration, est disposé de sorte à relier le bras 4 à une face interne 301 de la paroi 30 de l'enceinte réactionnelle 3, la face interne 301 étant en regard de la réaction 2. Et, de manière similaire, le joint 85 est disposé entre le soufflet 84 ou la poche cylindrique et la face interne 301 de la paroi 30.

Plus précisément, dans la figure 5A, le dispositif d'isolation hermétique 8 est extensible selon une course C dans l'enceinte réactionnelle 3.

En outre, lorsque le bras 4 translate selon le premier axe A1 de sorte à comprimer le dispositif d'isolation hermétique 8, comme représenté en figure 5B. Néanmoins, cette translation est limitée jusqu'à une position haute H1, position à laquelle le soufflet 84 ou la poche élastique ne peut pas être davantage comprimée.

Dès lors, il existe une zone morte M1 qui représente une zone pour laquelle le capteur 5 ne peut pas détecter d'information et observer le ou les paramètres à étudier.

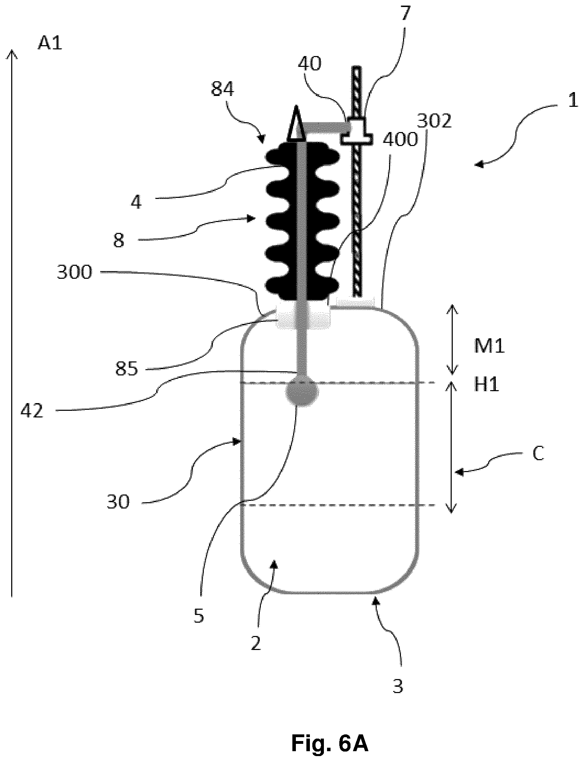

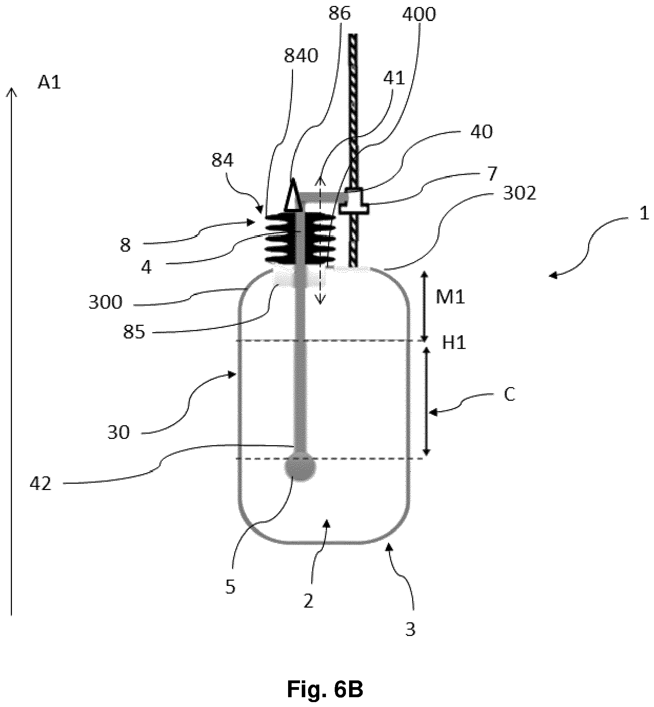

Ainsi, il peut être envisagé une deuxième configuration du dispositif d'analyse 1, comme représenté en figure 6A et en figure 6B.

Dans cette configuration, le dispositif d'isolation hermétique 8 est disposé à l'extérieur de l'enceinte réactionnelle 3. Plus précisément, le dispositif d'isolation hermétique 8 est disposé de sorte à relier le bras 4 à une face externe 302 de la paroi 30 de l'enceinte réactionnelle 3, la face externe 302 étant en regard d'un milieu extérieur à la réaction 2 et opposée à la face interne 301. Et, de manière similaire, le joint 85 est disposé entre le soufflet 84 ou la poche cylindrique et la face externe 302 de la paroi 30.

Cette deuxième configuration, avec le dispositif d'isolation hermétique 8 disposé à l'extérieur de l'enceinte réactionnelle 3, présente l'avantage d'augmenter la course C du bras 4 et donc de permettre au capteur 5 de pouvoir observer le ou les paramètres sur une plage de hauteur, dans l'exemple illustré, plus importante.

Afin de permettre au soufflet 84 ou à la poche élastique de conserver une forme naturelle lors des mouvements du bras 4, le soufflet 84 ou la poche élastique peut être couplé à un évent 86 permettant d'évacuer toute surpression à l'intérieur du dispositif d'isolation hermétique 8. L'évent 86 peut également être associé à un filtre 0,2 µm pour permettre une mise à pression atmosphérique sans laisser de contaminant pénétrer dans le dispositif d'isolation hermétique 8.

En outre, le dispositif d'analyse 1 selon l'invention présente l'avantage de ne nécessiter qu'un unique port de fixation, à savoir l'ouverture 400 au niveau de la face 300 de la paroi 30, pour l'insertion du bras 4 et du capteur 5 et d'être adapté pour un montage contre le port de fixation standard Clamp DN50 ou DN70 des enceintes réactionnelles 3 disponibles actuellement sur le marché. Les enceintes réactionnelles 3 standards ne nécessitent donc pas d'être adapté au dispositif d'analyse 1.

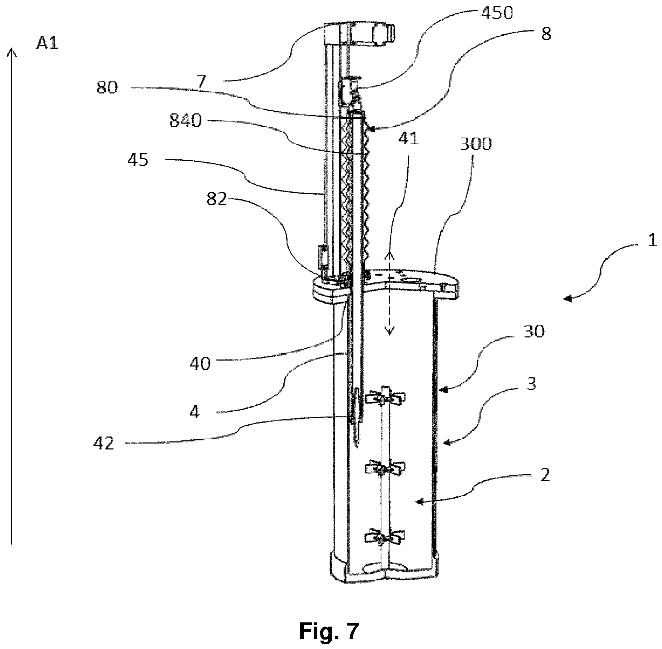

La figure 7 représente une vue détaillée du dispositif d'analyse et de suivi 1 d'au moins un paramètre de la réaction chimique ou biochimique ou biologique 2 dans l'enceinte réactionnelle 3 selon la configuration représentée en figure 6A et en figure 6B. Comme énoncé précédemment, le bras 4 a un degré de liberté en translation par rapport à la paroi 30 de l'enceinte réactionnelle 3 de sorte que le bras 4 translate sensiblement perpendiculairement par rapport à la face 300 de la paroi 30. Cette translation peut par exemple être opérée par le biais d'un guide de translation 45 reliant le dispositif de motorisation 7 et le bras 4. De manière préférentielle, le guide de translation 45 est un rail relié au bras au niveau d'une fixation 450. La fixation 450 est alors mobile sur le rail, à savoir le guide de translation 45, et le bras 4 y est fixé selon une liaison encastrement par exemple.

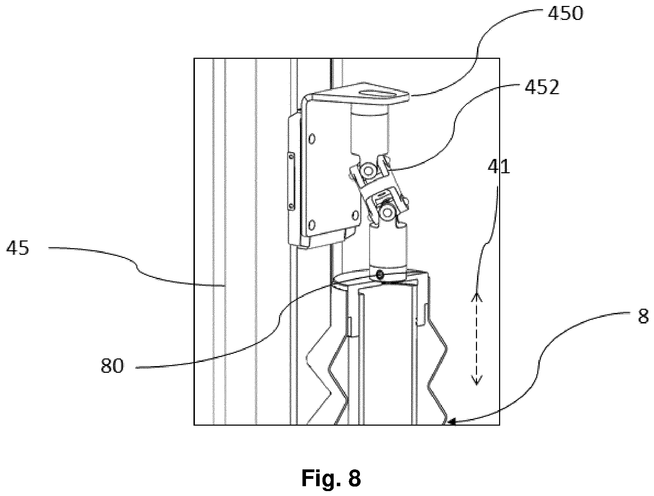

En variante, comme représenté en figure 8, la fixation 450 peut comprendre une liaison double cardan 452 assurant ainsi un bon alignement du bras 4 et permettant d'éviter tout porte à faux.

En outre, la fixation du capteur 5 à l'extrémité libre 42 du bras 4 peut également se faire par le biais d'une connectique souple et étanche comme par exemple une connectique de type PG13.5.

## Revendications

1. Dispositif d'analyse (1) d'au moins un paramètre d'une réaction (2) chimique ou biochimique ou biologique dans une enceinte réactionnelle (3), le dispositif d'analyse (1) comprenant:
- un bras (4) destiné à être fixé selon une liaison glissière (41) contre une face (300) d'une paroi (30) de l'enceinte réactionnelle (3) par l'intermédiaire d'une extrémité fixe (40), le bras (4) ayant un degré de liberté en translation par rapport à la paroi (30) de l'enceinte réactionnelle (3) ;
- un capteur (5) configuré pour mesurer l'au moins un paramètre, le capteur (5) étant fixé à une extrémité libre (42) du bras (4) ;
- un dispositif de motorisation (7) configuré pour induire un déplacement axial du bras (4) selon le degré de liberté ;
- un dispositif d'isolation hermétique (8) du bras (4) par rapport à la réaction (2) chimique ou biochimique ou biologique, le dispositif d'isolation hermétique (8) reliant le bras (4) à la face (300) de la paroi (30) de l'enceinte réactionnelle (3).

2. Dispositif d'analyse (1) selon la revendication 1, dans lequel le dispositif d'isolation hermétique (8) comprend un matériau parmi le silicone, un élastomère fluoré, le polyétheréthercétone (PEEK), l'éthylène-propylène-diène monomère (EPDM), du polytétrafluoroéthylène (PTFE).

3. Dispositif d'analyse (1) selon la revendication 1 ou 2, dans lequel le dispositif d'isolation hermétique (8) comprend un soufflet (84).

4. Dispositif d'analyse (1) selon l'une des revendications 1 à 3, dans lequel la paroi (30) comprend une face interne (301) en regard de la réaction (2), le dispositif d'isolation hermétique (8) étant disposé de sorte à relier le bras (4) à la face interne (301) de la paroi (30).

5. Dispositif d'analyse (1) selon l'une des revendications 1 à 3, dans lequel la paroi (30) comprend une face externe (302) en regard d'un milieu extérieur à la réaction (2), le dispositif d'isolation hermétique (8) étant disposé de sorte à relier le bras (4) à la face externe (302) de la paroi (30).

6. Dispositif d'analyse (1) selon l'une des revendications 1 à 5, comprenant un moyen de commande (9) du bras (4), connecté au dispositif de motorisation (7) et agencé pour commander le déplacement du bras (4) selon le degré de liberté.

7. Dispositif d'analyse (1) selon l'une des revendications précédentes, comprenant un moyen de transmission de données (6) susceptibles d'être mesurées par le capteur (5).

8. Dispositif d'analyse (1) selon l'une des revendications précédentes, comprenant un dispositif d'enregistrement (10) des données mesurées par le capteur (5).

9. Dispositif d'analyse (1) selon l'une des revendications précédentes, dans lequel le capteur (5) est un capteur apte à mesurer une donnée parmi le pH, l'oxygène dissout (O₂), le dioxyde de carbone (CO₂), la conductivité électrique, le potentiel redox ou la température.

10. Dispositif d'analyse (1) selon l'une des revendications précédentes, dans lequel le bras (4) est un bras télescopique.

11. Dispositif d'analyse (1) selon la revendication 3, dans lequel le dispositif d'isolation hermétique (8) comprend un joint (85) positionné entre le soufflet (84) et la face (300) de la paroi (30) de l'enceinte réactionnelle (3), le joint (85) comprenant une première surface d'étanchéité (850) en contact avec la face (300) de la paroi (30) de l'enceinte réactionnelle (3) et une deuxième surface d'étanchéité (852) en contact avec le soufflet (84).

12. Dispositif d'analyse (1) selon la revendication 11, dans lequel le joint (85) est un joint racleur.

13. Dispositif d'analyse (1) selon l'une des revendications précédentes, dans lequel le dispositif d'isolation hermétique (8) comprend un élément de guidage (88) configuré pour bloquer un mouvement relatif entre le bras (4) et la face (300) de la paroi (30) de l'enceinte réactionnelle (3) dans un plan parallèle à ladite face (300).

14. Enceinte réactionnelle (3) d'une réaction chimique ou biochimique ou biologique comprenant le dispositif d'analyse (1) d'au moins un paramètre de la réaction (2) chimique ou biochimique ou biologique dans l'enceinte réactionnelle (3) selon l'une quelconque des revendications précédentes.
